# EUROPEAN PATENT APPLICATION

(11) **EP 2 065 399 A1**
(43) Date of publication of application: **03.06.2009**
(21) Application number: 07122013.1
(22) Date of filing: 30.11.2007
(51) Int. Cl.: C07K 16/00, C12Q 1/68, G01N 33/574

(54) **The present invention relates to methods for prediction of the therapeutic success of breast cancer therapy**

(71) Applicant: Siemens Healthcare Diagnostics GmbH, 65760 Eschborn (DE)
(72) Inventor: Wirtz, Ralph, 50677, Köln (DE)
(74) Representative: Maier, Daniel Oliver

(57) **Abstract**

The present invention relates to a method for predicting a clinical response of a patient suffering from or at risk of developing gynecologic cancer towards a given mode of treatment, said method comprising the steps of:
a) obtaining a biological sample from said patient;
b) determining the expression level of at least one gene selected from the group comprising ALCAM, Osteopontin, Her-2/neu, EGFR, uPA/PAI-1 and/or ESR1, in particular of ALCAM and/or Osteopontin, in said sample;
c) comparing the pattern of expression level(s) determined in (b) with one or several reference pattern(s) of expression levels; and
d) predicting therapeutic success for said given mode of treatment in said patient or implementing therapeutic regimen in said patient from the outcome of the comparison in step (c).

## Description

### Field of the invention

The present invention relates to methods for prediction of the therapeutic success of breast cancer therapy

### Background of the invention

Every fourth cancer finding in women is breast cancer. Therewith, breast cancer is the most common cancer and the second most common cause of death among women in western industrial nations (Jemal et al., 2007) ¹. It is estimated that every eighth to tenth woman will develop breast cancer during her lifetime. With a total share of 10 % it is the third most common cancer worldwide (Veronesi et al., 2005) ². With an incidence of 130 cases per 100000 women there are about 55000 new cases in Germany annually from which 18000 cases will cause death (GEKID, 2006) ³.

Activated leukocyte cell adhesion molecule (ALCAM, other designations are MEMD and CD166) is a glycoprotein of the immunoglobulin superfamily of adhesion molecules mapped to human chromosome 3q13 (van Kempen et al., 2001 ⁴; Swart, 2002 ⁵). As cell surface immunoglobulin it is expressed in different kinds of normal and neoplastic tissues.

ALCAM has five extracellular immunoglobulin domains (two NH₂-terminal, membrane-distal variable- [V]-type folds and three membrane-proximal constant- [C2]-type immunoglobulin folds), a transmembrane region, and a short cytoplasmic tail (Swart, 2002 ⁵). It is involved in both homotypic/homophilic (to ALCAM) and heterotypic/heterophilic (to CD6) adhesions. ALCAM was identified on activated leukocytes, mesenchymal and hematopoietic stem cells, embryonal hematopoietic and endothelial cells, lung endothelial cells, endometrial cells, blastocysts etc. (Bruder et al., 1998 ⁶; Bowen and Aruffo, 1999 ⁷; Fujiwara et al., 2003 ⁸; Ohneda et al., 2001 ⁹; King et al., 2004 ¹⁰).

In neoplasms an altered ALCAM expression has been implicated in tumorigenesis and tumour progression of malignant melanoma (Swart et al., 2005 ¹¹), prostate cancer (Kristiansen et al., 2003 ¹²), colorectal carcinoma (Weichert et al., 2004 ¹³) and esophageal squamous cell carcinoma (Verma et al., 2005 ¹⁴) and very recently in breast carcinoma. In malignant melanoma ALCAM expression was reported to be significantly correlated with cancer progression and distinguishes the invasive and metastatic vertical growth phase (Swart et al., 2005 ¹¹). In prostate cancer, immunohistochemistry showed high ALCAM-levels in 81% and a decreased expression in 19% of cases. Compared with high-level expression, low-level expression occurred particularly in high-grade tumours. The authors suggested that downregulation of ALCAM protein expression might be involved in tumor progression and aggressive growth behaviour (Kristiansen et al., 2003 ¹²). In contrast to these findings ALCAM overexpression in colorectal carcinoma and esophageal squamous cell carcinoma was associated with late clinical stage and shortened patient survival (Weichert et al., 2004 ¹³; Verma et al., 2005 ¹⁴).

As for breast cancer, various studies have shown that ALCAM is present in many breast cancer cell lines and in resected breast tumours, but contradictory results have been published with respect of its prognostic value: By quantitative RT-PCR King et al. found that decreased ALCAM expression levels indicate a less differentiated phenotype and poor prognosis (King et al., 2004 ¹⁵). In an immunofluorescence study, Jezierska et al. confirmed these results by finding that low ALCAM expression correlated with an aggressive tumour phenotype (Jezierska et al., 2006 ¹⁶). In contrast, an immunohisto-chemical study in which membraneous and cytoplasmic staining were evaluated separately came to the result that high cytoplasmic ALCAM expression is an unfavorable prognostic indicator in univariate and multivariate analysis, especially in patients who received tamoxifen as adjuvant therapy (Burkhardt et al., 2006 ¹⁷).

Osteopontin (OPN, other designations are SSP1 and BNSP) is a phosphorylated glycoprotein involved in the regulation of bone development and immune system. It is secreted as well from a variety of tumor cells. Osteopontin interacts with a variety of cell surface receptors, including several integrins and CD44. So far, high OPN protein concentrations in tumor or in the blood of breast cancer patients have been associated with a poor prognosis (Tuck et al, 1998 ¹⁸; Rudland et al., 2002 ¹⁹; Bramwell et al., 2006 ²⁰), whereas in other studies no clear results could be obtained (Wang-Rodriguez et al., 2003 ²¹). A further study could demonstrate that OPN in mammacarcinoma stimulates the uPA-expression (Das et al., 2004 ²²).

It has been shown from several studies that the epidermal growth factor receptor (EGFR) plays an important role in cancer genesis. Said receptor, also named ErbB-1, is a cell-surface receptor for members of the epidermal growth factor family (EGF-family) of extracellular protein ligands. The epidermal growth factor receptor is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). It has been reported that mutations affecting EGFR expression or activity often result in cancer.

EGFR is a transmembrane protein receptor which is activated by binding of its specific ligands, including epidermal growth factor and transforming growth factor α (TGFα). Upon activation by its growth factor ligands, EGFR undergoes a transition from an inactive monomeric form to an active homodimer, which then stimuates cell growth, tissue proliferation and cell mitosis, the mechanism of which will be described in the following.

The said EGFR comprises a tyrsoine kinase on its intracellular domain. EGFR dimerization stimulates the activity of said tyrosine kinase. As a result, autophosphorylation of five tyrosine (Y) residues in the C-terminal domain of EGFR occurs. These are Y992, Y1045, Y1068, Y1148 and Y1173. This autophosphorylation elicits downstream activation and signaling by several other proteins that associate with the phosphorylated tyrosines through their own phosphotyrosine-binding SH2 domains. These downstream signaling proteins initiate several signal transduction cascades, principally the MAPK, AKt and JNK pathways, leading to DNA synthesis and cell proliferation ²³.

Such proteins modulate phenotypes such as cell migration, adhesion, and proliferation. The kinase domain of EGFR can also cross-phosphorylate tyrosine residues of other receptors it is aggregated with, and can itself be activated in that manner. One of the major effects of EGFR autophosphorylation is the upregulation of the expression of the Vascular Endothelial Growth Factor (VEGF), which, when being secreted, stimulates, among others, cell proliferation, particularly angiogenesis.

There is some evidence that in some cases preformed inactive dimers may also exist before ligand binding. In addition to forming homodimers after ligand binding, EGFR may pair with another member of the ErbB receptor family, such as ErbB2/Her-2/neu, to create an activated heterodimer. Moreover, there is evidence that in some cancerogenic cells the overexpression of EGFR leads to an elevated abundance of said receptor in the cellular membranes, which leads to autonomous dimerization due to high receptor density, without the need for the ligand to elicit said dimerization.

Hence, an overexpression of either native or mutant EGFR due is frequently found in cancerogenic and pre-cancerogenic cells and/or tissues. Said overexpression may be accompanied by mutations of the EGFR gene itself, as well as to gene amplification, polysomy, aneuploidy, genomic instability and the like. Said overexpression leads to a self-activation of cell proliferation in the respective cells and/or tissues due to autonomous dimerization, as well as to the enhanced secretion of VEGF, which in turn stimulates cell proliferation in the very same cells and/or tissues, as well as to an enhanced vascularization of the respective tissue due to an enhanced angiogenesis. Overexpression of EGFR does thus trigger a positive feedback mechanism which rapidly enforces tumor growth.

It is yet difficult to determine those tumor types which are promoted by EGFR overexpression. This is due to the fact that an overexpression rate of 2x, which is often enough to promote the above identified phenomena leading to increased malignancy of the tumor, can not be resolved with standard methods, i.e immunohistochemistry (IHC), fluorescent in situ hybridization (FISH) and/or quantitative PCR.

This means that, in tumor diagnosis, many tumor types which are characterized by enhanced EGFR expression and/or EGFR overexpression and are thus potential targets for EGFR inhibitors might remain undetected with standard methods. Hence, chances to treat these tumors with the most adequate treatments available to date are lost.

As EGFR is a member of the ErbB receptor family, it can be assumed that the above mentioned mechanism are also applicable to the other ErbB receptors introduced above.

As VEGFA is a member of the VEGF (Vascular endothelial growth factor) and related to the PDGF (Platelet derived growth factor) and FGF (Fibrobast growth factor) family, it can be assumed that the above mentioned mechanisms are also applicable to the other growth factors of said families.

As VEGFR1 is a member of the VEGFR (Vascular endothelial growth factor receptor) family, it can be assumed that the above mentioned mechanisms are also applicable to the other growth factors of said families, including the PDGFR and FGF Receptor family.

PAI-1 (plasminogen activator inhibitor-1) and uPA (urokinase plasminogen activator) are tumor biological prognostic factors in breast cancer. Breast cancer patients with high uPA and/or PAI-1 levels in their primary tumor tissue have a significantly lower chance for cure than patients with low levels of both uPA and PAI-1.

ESR 1 (estrogen receptor 1) is a ligand-activated transcription factor composed of several domains important for hormone binding, and activation of transcription. It is known that estrogen promotes the growth of some cancers, such as breast cancer, especially those that express ESR1.

Disease free survival (DFS) and overall survival (OAS) of high-risk breast cancer patients as determined by conventional clinical parameters (nodal status, grade, tumor size) is critical with far more than 20 tumor recurrence despite intensive treatment combined chemo- and endocrine therapy depending on risk stratification. However, molecular tests that better select a more appropriate therapy, for instance by adding targeted anti-cancer drugs, are not available yet.

Additionally, beside high costs, chemotherapy is often accompanied by severe side effects, and the response rate is far from satisfactory.

Therefore, there is a strong need for predictive factors for defining responsiveness to chemotherapy and for selecting a more appropriate therapy, respectively, such as a medication related to the signalling pathway of receptors from the ERB receptor family.

Definitions

The term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assessment of the malignancy of a tumor, or to the expected survival rate (DFS, disease free survival) of a patient, if the tumor remains untreated.

The term "clinical response" of a patient, as used herein, relates to the effectiveness of a certain therapy in a patient, meaning an improvement in any measure of patient status, including those measures ordinarily used in the art, such as overall survival, progression free survival, recurrence-free survival, and distant recurrence-free survival.

The term "neoplastic disease" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin).

The terms "cancer" and "cancerous" refer to or describe the physiological condition in mammals that is typically characterized by unregulated cell growth. The term "cancer" as used herein includes carcinomas, (e.g., carcinoma in situ, invasive carcinoma, metastatic carcinoma) and pre-malignant conditions, neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer and primary carcinomas are included.

As used herein, the term "gynecologic cancers" refers to cancer which are diagnosed in female breast and reproductive organs that include the uterus, ovaries, cervix, fallopian tubes, vulva, and vagina. Examples of gynecologic cancers include, but are not limited to breast cancer, ovarian cancer, vulvar cancer, vaginal cancer, tubal cancer, endometrian cancer and/or cervical cancer. As used herein, the term "endometrian cancer", also called endometrial cancer or uterine cancer, includes malignant growth of cells in the endometrium, the lining of the uterus.

The term "tumor" as used herein, refers to all neoplastic cell growth and proliferation, whether malignant or benign, and all pre-cancerous and cancerous cells and tissues.

The term "determining the status" as used herein, refers to a measurable property of a gene and its products, especially on the nucleotide level and the gene level including mutation status and gene expression status. A number of parameters to determine the status of a gene and its products can be used including, but not limited to, determining the level of protein expression, the amplification or expression status on RNA level or DNA level, of polynucleotides and of polypeptides, and the analysis of haplotype or the mutation status of the gene. An exemplary determinable property correlated with the status of estrogen receptor or progesterone receptor is the amount of the estrogen receptor or progesterone receptor RNA, DNA or other polypeptide in the sample or the presence of nucleotide polymorphisms.

The terms "biological sample", as used herein, refer to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, or cells there from. Biological samples may also include sections of tissues such as frozen or fixed sections taken for histological purposes or microdissected cells or extracellular parts thereof. A biological sample to be analyzed is tissue material from neoplastic lesion taken by aspiration or punctuation, excision or by any other surgical method leading to biopsy or resected cellular material. Such biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

The term "therapy modality", "regimen" or as well as "therapeutic regimen" refers to a timely sequential or simultaneous administration of anti-tumor, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, time-frame of application and frequency of administration within a defined therapy window. Currently various combinations of various drugs and/or physical methods, and various schedules are under investigation.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

The term "small molecule", as used herein, is meant to refer to a compound which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The terms "regulated" or "regulation" and "differentially regulated" as used herein refer to both upregulation [i.e., activation or stimulation (e.g., by agonizing or potentiating] and down regulation [i.e., inhibition or suppression (e.g., by antagonizing, decreasing or inhibiting)].

The term "transcriptome" relates to the set of all messenger RNA (mRNA) molecules, or "transcripts", produced in one or a population of cells. The term can be applied to the total set of transcripts in a given organism, or to the specific subset of transcripts present in a particular cell type. Unlike the genome, which is roughly fixed for a given cell line (excluding mutations), the transcriptome can vary with external environmental conditions. Because it includes all *mRNA* transcripts in the cell, the transcriptome reflects the genes that are being actively expressed at any given time, with the exception of mRNA degradation phenomena such as transcriptional attenuation. The discipline of transcriptomics examines the expression level of mRNAs in a given cell population, often using high-throughput techniques based on DNA microarray technology.

The term "expression levels" refers, e.g., to a determined level of gene expression. The term "pattern of expression levels" refers to a determined level of gene expression compared either to a reference gene (e.g. housekeeper or inversely regulated genes) or to a computed average expression value (e.g. in DNA-chip analyses). A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, endothelial cells of blood vessels as well as cells of the immune system (e.g. lymphocytes, macrophages, killer cells).

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

"Primer pairs" and "probes", within the meaning of the invention, shall have the ordinary meaning of this term which is well known to the person skilled in the art of molecular biology. In a preferred embodiment of the invention "primer pairs" and "probes", shall be understood as being polynucleotide molecules having a sequence identical, complementary, homologous, or homologous to the complement of regions of a target polynucleotide which is to be detected or quantified. In yet another embodiment nucleotide analogues are also comprised for usage as primers and/or probes.

"Arrayed probes", within the meaning of the invention, shall be understood as being a collection of immobilized probes, preferably in an orderly arrangement. In a preferred embodiment of the invention, the individual "arrayed probes" can be identified by their respective position on the solid support, e.g., on a "chip".

The phrase "therapeutic success" refers, in the adjuvant chemotherapeutic setting to the observation of a defined tumor free or recurrence free survival time (e.g. 2 years, 4 years, 5 years, 10 years). This time period of disease free survival may vary among the different tumor entities but is sufficiently longer than the average time period in which most of the recurrences appear. In a neo-adjuvant therapy modality, response may be monitored by measurement of tumor shrinkage due to apoptosis and necrosis of the tumor mass.

The term "recurrence" includes distant metastasis that can appear even many years after the initial diagnosis and therapy of a tumor, or local events such as infiltration of tumor

cells into regional lymph nodes, or occurrence of tumor cells at the same site and organ of origin within an appropriate time.

"Prediction of therapeutic success" does refer to the methods described in this invention. Wherein a tumor specimen is analyzed for it's gene expression and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prolonged disease free post therapy time.

The term "marker" refers to a biological molecule, e.g., a nucleic acid, peptide, protein, hormone, etc., whose presence or concentration can be detected and correlated with a known condition, such as a disease state.

The term "ligand", as used herein, relates to a molecule that is able to bind to and form a complex with a biomolecule to serve a biological purpose. In a narrower sense, it is an effector molecule binding to a site on a target protein, by intermolecular forces such as ionic bonds, hydrogen bonds and Van der Waals forces. The docking (association) is usually reversible (dissociation). Actual irreversible covalent binding between a ligand and its target molecule is rare in biological systems. Ligand binding to receptors often alters the chemical conformation, i.e. the three dimensional shape of the receptor protein. The conformational state of a receptor protein determines the functional state of a receptor. The tendency or strength of binding is called affinity. Ligands include substrates, inhibitors, activators, and neurotransmitters.

The term "agonist", as used herein, relates to a substance that binds to a specific receptor and triggers a response in the cell. It mimics the action of an endogenous ligand that binds to the same receptor.

The term "receptor", as used herein, relates to a protein on the cell membrane or within the cytoplasm or cell nucleus that binds to a specific molecule (a ligand), such as a neurotransmitter, hormone, or other substance, and initiates the cellular response to the ligand. Ligand-induced changes in the behavior of receptor proteins result in physiological changes that constitute the biological actions of the ligands.

The term "signalling pathway" is related to any intra- or intercellular process by which cells converts one kind of signal or stimulus into another, most often involving ordered sequences of biochemical reactions out- and inside the cell, that are carried out by enzymes and linked through homones and growth factors (intercellular), as well as second messengers (intracellular), the latter resulting in what is thought of as a "second messenger pathway". In many signalling pathways, the number of proteins and other molecules participating in these events increases as the process eminates from the initial stimulus, resulting in a "signal cascade" and often results in a relatively small stimulus eliciting a large response.

The term "marker gene," as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and head and neck, colon or breast cancer in particular. A marker gene may also have the characteristics of a target gene.

"Target gene", as used herein, refers to a differentially expressed gene involved in breast cancer in a manner in which modulation of the level of the target gene expression or of the target gene product activity may act to ameliorate symptoms of breast cancer. A target gene may also have the characteristics of a marker gene.

The term "expression level", as used herein, relates to the process by which a gene's DNA sequence is converted into functional protein and particularly to the amount of said conversion.

When used in reference to a single-stranded nucleic acid sequence, the term "substantially homologous" refers to any probe that can hybridize (i.e., it is the complement of) the single-stranded nucleic acid sequence under conditions of low stringency as described above.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described below. When it comes to the determination of expression levels, hybridization based methods may for example be used to determine the amount of mRNA for a given gene.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR)

The term "determining the protein level", as used herein, refers to methods which allow the quantitative and/or qualitative determination of one or more proteins in a sample. These methods include, among others, protein purification, including ultracentrifugation, precipitation and chromatography, as well as protein analysis and determination, including the use protein microarrays, two-hybrid screening, blotting methods including western blot, one- and two dimensional gelelectrophoresis, isoelectric focusing and the like.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

### Object of the invention

It is one object of the present invention to provide an improved method for the prediction of a clinical response of a patient suffering from or at a risk of breast cancer towards an agent to current status tests.

It is yet another object of the present invention to provide a method for the determination whether or not a tumor is likely to be susceptible to chemotherapy and a more appropriate therapy, respectively, in particular to a medication related to the signalling pathway of receptors from the ERB receptor family. In a preferred embodiment this refers to therapies targeting ERB receptor family members itself. In yet another embodiment this refers to gene products regulated by ERB family members and downstream activities thereof, such as family members of the VEGF/VEGFR and/or FGF/FGFR system.

These objects are met with methods and means according to the independent claims of the present invention. The dependent claims are related to preferred embodiments. It is yet to be understood that value ranges delimited by numerical values are to be understood to include the said delimiting values.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to the invention, a method is provided for predicting a clinical response of a patient suffering from or at risk of developing a gynecologic cancer, preferably breast cancer, towards a given mode of treatment, said method comprising the steps of:
a) obtaining a biological sample from said patient;
b) determining the expression level of at least one gene selected from the group comprising ALCAM, Osteopontin, Her-2/neu, EGFR, uPA/PAI-1 and/or ESR1, in particular of ALCAM and/or Osteopontin, in said sample;
c) comparing the pattern of expression level(s) determined in (b) with one or several reference pattern(s) of expression levels; and
d)predicting therapeutic success for said given mode of treatment in said patient or implementing therapeutic regimen in said patientfrom the outcome of the comparison in step (c).

In a preferred embodiment of the present invention, it is provided that the method is characterized in that
a) upregulated expression of ALCAM is indicative of a good prediction as regards therapeutic success for patients receiving a chemotherapy,
b) downregulated expression of ALCAM is indicative of a poor prediction as regards therapeutic success for patients receiving a chemotherapy,
c) upregulated expression of Osteopontin, Her-2/neu, EGFR and/or ESR1 is indicative of a poor prediction as regards therapeutic success for patients receiving a chemotherapy, and/or
d) downregulated expression of Osteopontin, Her-2/neu, EGFR and/or ESR1 is indicative of a good prediction as regards therapeutic success for patients receiving a chemotherapy.

In another preferred embodiment of the present invention, it is provided that upregulated expression of Osteopontin and/or EGFR and/or downregulated expression of ALCAM, Her-2/neu, uPA/PAI-1 and/or ESR1 is indicative of a promising prediction as regards therapeutic success for patients receiving a therapeutic regimen targeting the signalling pathway of receptors from the VEGF receptor and /or ligand family.

In another preferred embodiment of the present invention, it is provided that the mode of treatment for which prediction is sought is a treatment related to the signalling pathway of receptors from the ErbB receptor family.

The ErbB family of receptors, comprises four closely related receptor tyrosine kinases, namely
- EGFR (ErbB-1) (epidermal growth factor receptor), also known as ErbB-1 or HER1
- HER2 (ErbB-2; Her-2/neu),
- HER3 (ErbB-3), and
- HER4 (ErbB-4).

There is evidence that any of these receptors may be related to cancer genesis, as well as to an enhanced expression of VEGF factors. However, a preferred receptor of the method according to the invention is EGFR, as there is
- a number of medicaments available which relate to the EGFR signalling pathway,
- the interaction between EGFR and VEGF expression has been clearly demonstrated,
- the role of EGFR in tumor genesis is well known, and
- the therapeutic effect of tumor medication directed to the EGFR signalling pathway has been demonstrated.

The PDGF family of growth factors comprises several members which all have in common that they feature a cystine-knot domain, and bind to tyrosine kinase receptors, like those from the ErbB family. The VEGF family comprises members of the VEGF sub-family, i.e.
- VEGF-A,
- VEGF-B,
- VEGF-C,
- VEGF-D.
and the Placenta growth factor (PlGF), as well as Platelet derived growth factors (PDGF-A and PDGF-B).

A number of other VEGF-related proteins have also been discovered encoded by viruses (VEGF-E) and in the venom of some snakes (VEGF-F).

All of these growth factors are ligands which are related to the ErbB signalling pathway, as their expression level is upregulated upon activation or self activation of a receptor of the ErbB family, particularly of EGFR. The growths factors do thus meet the above identified definition according to which the said ligand is related to the signalling pathway of receptors from the ErbB receptor family.

In another preferred embodiment of the present invention, it is provided that the mode of treatment for which prediction is sought is a treatment related to the signalling pathway of receptors from the VEGF receptor and/or ligand family.

In yet another preferred embodiment of the present invention, it is provided that said given mode of treatment (a) acts on recruitment of lymphatic vessels, cell proliferation, cell survival and/or cell motility, and/or b) comprises administration of a chemotherapeutic agent.

Furthermore, it is provided an another preferred embodiment of the present invention that said given mode of treatment comprises chemotherapy, administration of small molecule inhibitors, antibody based regimen, anti-proliferation regimen, pro-apoptotic regimen, pro-differentiation regimen, radiation and/or surgical therapy.

In another embodiment of the present invention, method of selecting a therapy modality for a patient afflicted with breast cancer is provided, said method comprising the steps of:
a) obtaining a biological sample from said patient;
b) predicting from said sample, by the method according to any one of the above methods, therapeutic success for a plurality of individual modes of treatment; and
c) selecting a mode of treatment which is predicted to be successful in step (b).

The inventors of the present invention have provided an improved method for identifying different collectives in a cohort of breast cancer patients. Due to the strongly upregulated mRNA expressions of ESR1 and Her-2/neu in breast cancer patients, it is very difficult to identify different collectives. Therefore, the inventors of the present invention have for the first time suggested to divide the mRNA expression data of the dominant marker genes ESR1 and Her-2/neu by a factor 5. Thus, the inventors of the present invention were for the first time in a position to identify by said method a high-risk collective via a suitable adjustment of the mRNA expression data.

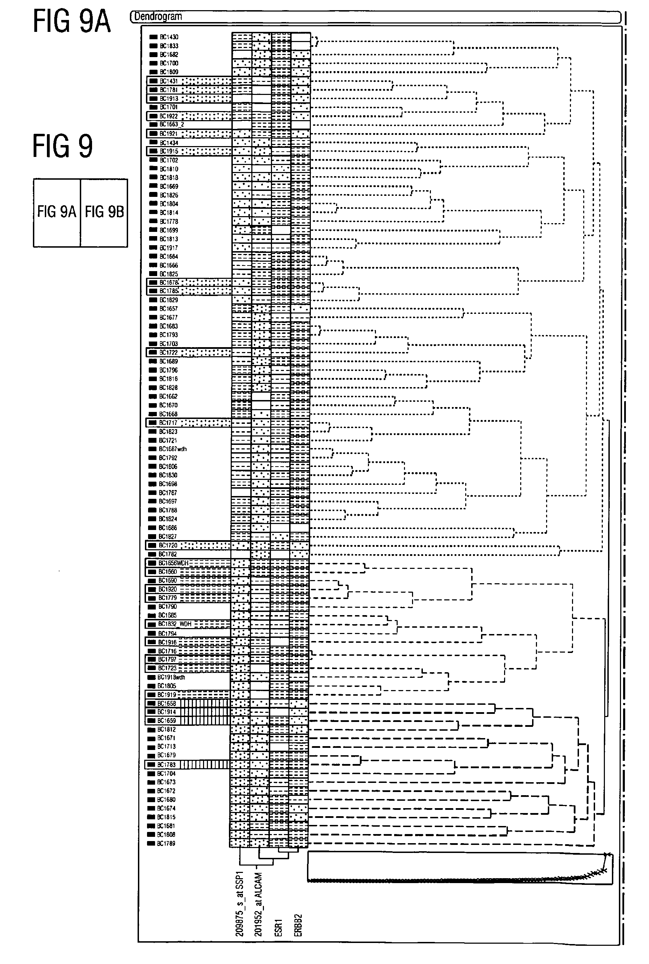

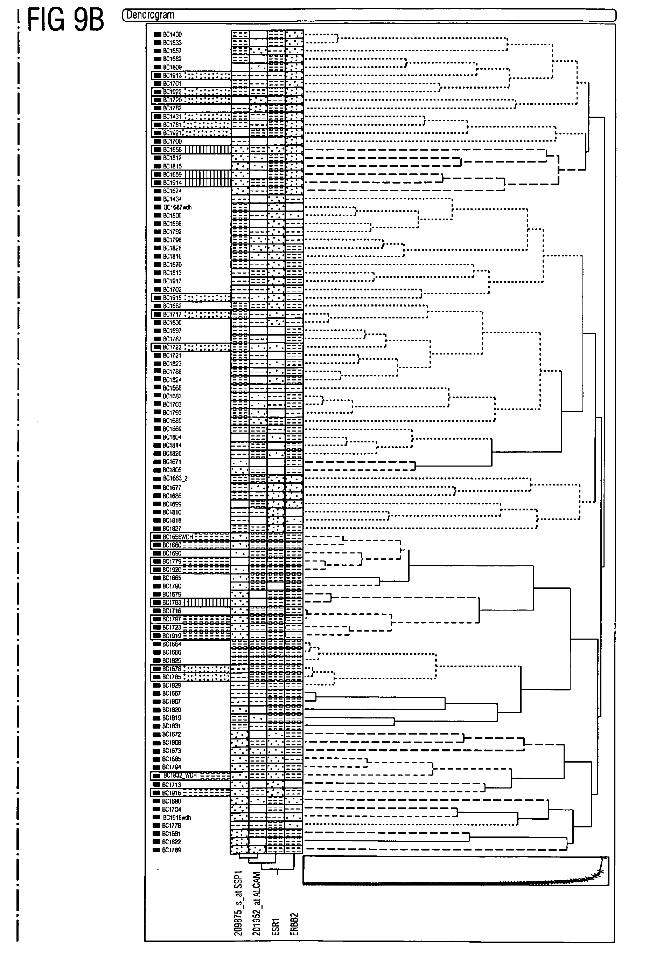

The patients of said high-risk collective have a poor prediction towards chemotherapy, but have a promising prediction towards therapeutic regimen targeting the signalling pathway of receptors from the VEGF receptor and/or ligand family. The inventors of the present invention could identify by clustering the mRNA expression data (OAS) of Osteopontin, ALCAM, ESR1 and Her-2/neu said high-risk collective. Via Figure 9 the improved results of the adjusted dendrogram (left dendrogram, the high-risk collective is the second collective from the bottom) compared to the worse results of a not adjusted dendrogram (right dendrogram) are demonstrated.

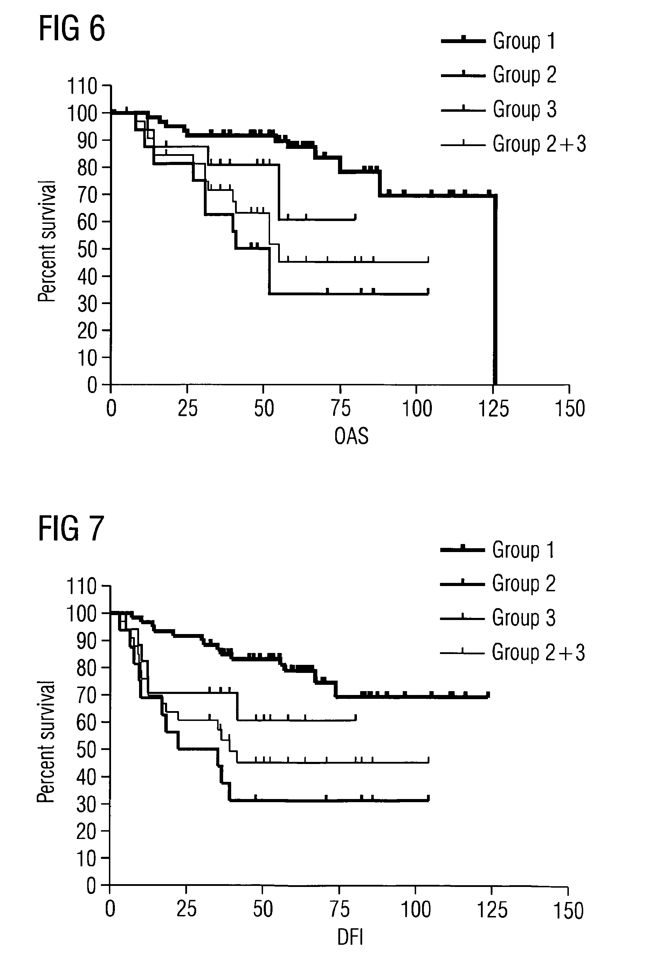

As mentioned already, it is yet difficult to determine those tumor types which are promoted by EGFR overexpression. Therefore, there exists a strong need for an alternative method for the determination whether or not a tumor is likely to be susceptible to a medication related to the signalling pathway of receptors from the ERB receptor family. The inventors of the present invention have for the first time suggested not to use said targets themselves as marker genes, but ALCAM and/or Osteopontin with or without combination of Her-2/neu, EGFR, uPA/PAI-1 and/or ESR1 as marker genes to predict the clinical response of a patient suffering from or at risk of developing breast cancer towards a medicament related to the signalling pathway of receptors from the ErbB receptor family. Said high-risk collective is characterized by upregulated expression of Osteopontin and EGFR and downregulated expression of ALCAM, Her-2/neu, uPA/PAI-1 and ESR1. Said collective newly identified in the present invention has a very poor prognosis and is depicted as group 2 in Figures 6 to 8 of example 2.

In another embodiment of the present invention, method of determining the prognosis for a patient afflicted with gynecologic cancer, preferably breast cancer, and receiving chemotherapy is provided, said method comprising the steps of:
a) obtaining a biological sample from said patient;
b) combining the expression level data of at least two genes selected from the group comprising ALCAM, Osteopontin, Her-2/neu, EGFR, uPA/PAI-1 and/or ESR1 in said sample;
c) comparing the pattern of expression level(s) determined in (b) with one or several reference pattern(s) of expression levels; and
d) determining the prognosis in said patient from the outcome of the comparison in step (c).

The inventors of the present invention have for the first time suggested to combine the expression level data of said marker genes to determine the prognosis for said patients. Due to this new approach the prognosis is more sensitive and more specific compared to current status tests with regard to patients receiving chemotherapy.

In another preferred embodiment of the present invention, it is provided that
a) upregulated expression of ALCAM is indicative of a good prognosis for patients receiving a chemotherapy,
b) downregulated expression of ALCAM is indicative of a poor prognosis for patients receiving a chemotherapy,
c) upregulated expression of Osteopontin, Her-2/neu, EGFR and/or ESR1 is indicative of a poor prognosis for patients receiving a chemotherapy, and/or
d) downregulated expression of Osteopontin, Her-2/neu, EGFR and/or ESR1 is indicative of a good prognosis for patients receiving a chemotherapy.

In another preferred embodiment of the present invention, it is provided that determining the expression level comprises:
a) determining the RNA expression level; and/or
b) determining the protein expression level.

It is particularly preferred that, in the method according to the invention, the said expression level is determined by
a) a hybridization based method;
b) a PCR based method;
c) determining the protein level, and/or by
d) an array based method.

In yet another preferred embodiment of the present invention, it is provided that the expression level of at least one of the said marker genes is determined with rtPCR (reverse transcriptase polymerase chain reaction) of the related mRNA.

In this preferred embodiment, the mRNA related to the markers is determined, namely with a reverse transcriptase polymerase chain reaction approach.

The inventors of the present invention have surprisingly found out that the determination of the marker genes' mRNA levels is very informative for the prediction of the clinical response of a patient suffering from or at risk of developing breast cancer towards a medicament.

In this context, it seems much more promising to provide anti-ErbB/anti-EGFR treatments, if so indicated, than anti-PDGF/anti-VEGF treatments (for example the anti-VEGF antibody Bevacizumab), because, in the signalling pathway, ErbB/EGFR is located upstream of PDGF/VEGF. This means that, if ErbB/EGFR is inhibited, a number of processes located downstream in the signalling pathway may be suppressed, among which VEGF-induced processes are only one example.

VEGF-related processes may be considered as one symptom out of a range of several symptoms related to tumor genesis, whereas defects in EGFR expression may be considered as the underlying cause for these symptoms, the treatment of which seems thus to be much more promising than the treatment of just one of said symptoms.

In addition, it can be concluded that combined anti-ErbB/anti-EGFR treatments and anti-PDGF/anti-VEGF treatments may be most promising in this situation.

In another preferred embodiment of the present invention, it is provided that the expression level of at least one of the said marker genes is determined in formalin and/or paraffin fixed tissue samples.

Routinely, in tumor diagnosis tissue samples are taken as biopsies form a patient and undergo diagnostic procedures. For this purpose, the samples are fixed in formalin and/or paraffin and are then examined with immunohistochemistry methods. The formalin treatment leads to the inactivation of enzymes, as for example the ubiquitous RNA-digesting enzymes (RNAses). For this reason, the mRNA status of the tissue (the so called transcriptome), remains undigested.

However, the formalin treatment leads to partial depolymerization of the individual mRNA molecules. For this reason, the current doctrine is that formalin fixed tissue samples can not be used for the analysis of the transcriptome of said tissue.

For this reason, it is provided in a preferred embodiment of the present invention that after lysis, the samples are treated with silica-coated magnetic particles and a chaotropic salt, in order to purify the nucleic acids contained in said sample for further determination.

Collaborators of the inventors of the present invention have developed an approach which however allows successful purification of mRNA out of tissue samples fixed in such manner, and which is disclosed, among others, in WO03058649, WO2006136314A1 and DE10201084A1, the content of which is incorporated herein by reference.

Said method comprises the use of magnetic particles coated with silica (SiO₂). The silica layer is closed and tight and is characterized by having an extremely small thickness on the scale of a few nanometers. These particles are produced by an improved method that leads to a product having a closed silica layer and thus entail a highly improved purity. The said method prevents an uncontrolled formation of aggregates and clusters of silicates on the magnetite surface whereby positively influencing the additional cited properties and biological applications. The said magnetic particles exhibit an optimized magnetization and suspension behavior as well as a very advantageous run-off behavior from plastic surfaces. These highly pure magnetic particles coated with silicon dioxide are used for isolating nucleic acids, including DNA and RNA, from cell and tissue samples, the separating out from a sample matrix ensuing by means of magnetic fields. These particles are particularly well-suited for the automatic purification of nucleic acids, mostly from biological body samples for the purpose of detecting them with different amplification methods.

The selective binding of these nucleic acids to the surface of said particles is due to the affinity of negatively charged nucleic acids to silica containing media in the presence of chaotropic salts like guanidinisothiocyanate. Said binding properties are known as the so called "boom principle". They are described in the European patent EP819696.

The said approach is particularly useful for the purification of mRNA out of formalin and/or paraffin fixed tissue samples. In contrast to most other approaches, which leave very small fragments behind that are not suitable for later determination by PCR and/or hybridization technologies, the said approach creates mRNA fragments which are large enough to allow specific primer hybridization and/or specific probe hybridization. A minimal size of at least 100 bp, more preferably 200 base pairs is needed for specific and robust detection of target gene expression. Moreover it is also necessary to not have too many inter-sample variations with regard to the size of the RNA fragments to guarantee comparability of gene expression results. Other issues of perturbance of expression data by sample preparation problems relate to the contamination level with DNA, which is lower compared to other bead based technologies. This of particular importance, as the inventors have observed, that DNAse treatment is not efficient in approximately 10% of FFPE samples generated by standard procedures and stored at room temperature for some years before cutting and RNA extraction.

The said approach thus allows a highly specific determination of candidate gene expression levels with one of the above introduced methods, particularly with hybridization based methods, PCR based methods and/or array based methods, even in formalin and/or paraffin fixed tissue samples, and is thus extremely beneficial in the context of the present invention, as it allows the use of tissue samples fixed with formalin and/or paraffin, which are available in tissue banks and connected to clinical databases of sufficient follow-up to allow retrospective analysis.

In another preferred embodiment of the present invention, it is provided that said treatment related to the signalling pathway of receptors from the ErbB receptor family comprises the administration of an agonist of the ErbB receptor domain.

In yet another preferred embodiment said treatment related to the signalling pathway of receptors from the ErbB receptor family comprises the administration of nucleotides, ribozomes, aptamers and /or nucleotide analogues capable of affecting the expression of ErbB receptor, VEGF receptor and /or VEGFA ligand expression. Preferably these substances act via downregulation of mRNA levels of said candidate genes. However, also induction or elevation of gene expression by introduction of receptor gene expression (e.g. ERBB-4 and/or VEGFD expression) can be advantageous to counteract the tumorpromoting activity (e.g. of EGFR, Her-2/neu and/or VEGFC).

By way of illustration and not by way of restriction said agonists may be selected from the group consisting of Cetuximab (tradename Erbitux®, target receptor is EGFR), Matuzumab (EMD7200, target receptor is EGFR), Trastuzumab (tradename Herceptin®, target receptor is HER2/neu), Pertuzumab (target receptor is HER2/neu), Bevacizumab (tradename Avastin®, target ligand is VEGFA), 2C3 (target ligand is VEGFA), VEGF-trap (AVE-0005, target ligands are VEGFA and PIGF), IMC-1121B (target receptor is VEGFR2), and CDP-791 (target receptor is VEGFR2).

In yet another preferred embodiment said treatment related to the signalling pathway of receptors from the ErbB receptor family comprises the administration of a tyrosin kinase inhibitor.

By way of illustration and not by way of restriction said inhibitors may be seleccted from the group consisting of Gefitinib (tradename Iressa®, ZD-1839, target receptor is EGFR), Eriotinib (tradename Tarceva®, OSI-774, target receptor is EGFR), EKB-569 (target receptor is EGFR), PKI-166 (target receptor is EGFR),), PKI-166 (target receptor is EGFR), Lapatinib (tradename tycerb®, target receptor is EGFR and Her-2/neu), GW572016 (target receptors are EGFR and Her-2/neu), AEE-788 (target receptors are EGFR, Her-2/neu and VEGFR-2), CI-1033 (target receptors are EGFR, Her-2/neu and Her4), and AZD6474 (target receptors are EGFR and VEGFR-2).

However, other treatments related to the ErbB receptor family signalling pathway which fall under the scope of the present invention comprise the administration of Sorafenib (tradename Nexavar®, BAY 43-9005, target receptors are VEGFR-2, VEGFR-3, c-KIT, PDGFR-B, RET and Raf-Kinase), BAY 57-9352 (target receptor is VEGFR-2), Sunitinib (tradename Sutent®, target receptors are VEGFR-1, VEGFR-2 and PDGFR), AG13925 (target receptors are VEGFR-1 and VEGFR-2), AG013736 (target receptors are VEGFR-1 and VEGFR-2), AZD2171 (target receptors are VEGFR-1 and VEGFR-2), ZD6474 (target receptors are VEGFR-1, VEGFR-2 and VEGFR-3), PTK-787/ZK-222584 (target receptors are VEGFR-1 and VEGFR-2), CEP-7055 (target receptors are VEGFR-1, VEGFR-2 and VEGFR-3), CP-547 (target receptors are VEGFR-1 and VEGFR-2), CP-632 (target receptors are VEGFR-1 and VEGFR-2), GW786024 (target receptors are VEGFR-1, VEGFR-2 and VEGFR-3), AMG706 (target receptors are VEGFR-1, VEGFR-2 and VEGFR-3), Imatinib mesylate (tradename Glivec®/Gleevec®, target receptors are bcr-abl and c-KIT), BMS-214662 (target enzyme is Ras farnesyl transferase), CCI-779 (target enzyme is mTOR), RAD0001 (tradename everolismus®, target enzyme is mTOR), CI-1040 (target enzyme is MEK), SU6668 (target receptors are VEGFR-2, PDGFR-B and FGFR-1), AZD6126, CP547632 (target receptors are VEGFRs), CP868596 GW786034 (target receptors are PDGFRs), ABT-869 (target receptors are VEGFRs and PDGFRs), AEE788 (target receptors are VEGFRs and PDGFRs), AZD0530 (target enzymes are src and abl), and CEP7055.

In yet another preferred embodiment the treatment comprises the administration of chemotherapeutics.

Said chemotherapeutics may be selected from the group consisting of Cyclophosphamid (Endoxan®, Cyclostin®). Adriamycin (Doxorubicin) (Adriblastin®), BCNU (Carmustin) (Carmubris®), Busulfan (Myleran®),Bleomycin (Bleomycin®), Carboplatin (Carboplat®), Chlorambucil (Leukeran®), Cis-Platin (Cisplatin®), Platinex (Platiblastin®), Dacarbazin (DTIC®; Detimedac®), Docetaxel (Taxotere®), Epirubicin (Farmorubicin®), Etoposid (Vepesid®), 5-Fluorouracil (Fluroblastin®, Fluorouracil®), Gemcitabin (Gemzar®), Ifosfamid (Holoxan®), Interferon alpha (Roferon®), Irinotecan (CPT 11, Campto®), Melphalan (Alkeran®),Methotrexat (Methotrexat®, Farmitrexat®), Mitomycin C (Mitomycin®), Mitoxantron (Novantron®), Oxaliplatin (Eloxatine®), Paclitaxel (Taxol®), Prednimustin (Sterecyt®), Procarbazin (Natulan®), Ralitrexed (Tomudex®), Trofosfamid (Ixoten®), Vinblastin (Velbe®), Vincristin (Vincristin®), Vindesin (Eldisine®), Vinorelbin (Navelbine®).

Furthermore, a kit useful for carrying out one of the said methods, comprising at least
a) a primer pair and/or a probe each having a sequence sufficiently complementary to a gene encoding for ALCAM, Osteopontin, Her-2/neu, EGFR, uPA/PAI-1 and/or ESR1; and/or
b) an antibody directed against ALCAM, Osteopontin, Her-2/neu, EGFR, uPA/PAI-1 and/or ESR1
is provided.

Furthermore, use of
a) an antibody directed against a ligand from the VEGF family,
b) an antisense nucleic acid or a ribozyme inhibiting the expression of a gene encoding for a ligand from the VEGF family, or
c) an inactive version of a ligand from the VEGF family
as an antagonist for the preparation of a pharmaceutical composition for the treatment of said high-risk breast cancer patients identified by a method according to the present invention is provided.

In yet another embodiment of the invention a method for correlating the clinical outcome of a patient suffering from or at risk of developing a gynecologic cancer, preferably breast cancer, with the presence or non-presence of a defect in expression of at least one gene selected from the group comprising ALCAM, Osteopontin, Her-2/neu, EGFR, uPA/PAI-1 and/or ESR1 is provided, said method comprising the steps of:
a) obtaining a (fixed) biological sample from said patient;
b) determining the expression level of at least one of the said marker genes in said patient according to any of the above methods; and
c) correlating the pattern of expression level(s) determined in (b) with said patient's data, said data being selected from the group consisting of etiopathology data, clinical symptoms, anamnesis data and/or data concerning the therapeutic regimen.

The said method is particularly beneficial for epidemiological studies. These studies profit from the fact that large tissue databases exist comprising paraffin and/or formalin fixed tissue samples together with an extensive documentation of the patient's history, including etiopathology data, clinical symptoms, anamnesis data and/or data concerning the therapeutic regimen.

The said methods allow for large scale studies which comprise the correlation of the clinical outcome of a patient suffering from or at risk of developing breast cancer with the presence or non-presence of a defect in expression of at least one of the marker genes according to the present invention. In order to successfully adopt this approach, the above introduced method for mRNA purification comprising silica coated magnetic beads and chaotropic salts is quite helpful.

Furthermore, a nucleic acid primer pair and/or a nucleic acid probe having a sequence sufficiently complementary to a gene encoding for ALCAM, Osteopontin, Her-2/neu, EGFR, uPA/PAI-1 and/or ESR1 is provided.

Genes of interest are listed in Table 4.

An antibody directed against ALCAM, Osteopontin, Her-2/neu, EGFR, uPA/PAI-1 and/or ESR1 is provided.

In a yet preferred embodiment of the present invention said gynecologic cancer is selected from the group comprising breast cancer, ovarian cancer, vulvar cancer, vaginal cancer, tubal cancer, endometrian cancer and/or cervical cancer.

In an especially preferred embodiment of the present invention said gynecologic cancer is breast cancer. The method according to the invention may be used for the analysis of a wide variety of neoplastic cell growth and proliferation of the breast tissues including, but not limited to ductal carcinoma in situ, lobular carcinoma, colloid carcinoma, tubular carcinoma, medullary carcinoma, metaplastic carcinoma, intraductal carcinoma in situ, lobular carcinoma in situ and papillary carcinoma in situ.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

### Example 1

### Patients

For analysis of ALCAM protein expression, primary breast cancer tissue from 160 patients mainly diagnosed at early stages were collected after surgery, snap-frozen and stored in liquid nitrogen. For array-based ALCAM mRNA detection, samples from 162 cases were analysed, with an overlap of 133 cases between both cohorts. All patients were treated for breast cancer at the University Clinics Hamburg Eppendorf, Germany, Department of Gynecology between 1992-2002. Before operation, informed consent for the scientific use of tissue materials was obtained from the patients. The study was performed in accordance with the principles of the declaration of Helsinki after approval by the local ethics committee.
No therapy like radiotherapy or neoadjuvant chemotherapy had been performed prior to operation. Breast conserving surgery was performed in 53% of patients, 47% were treated by mastectomy. All additional histological and clinical tumor characteristics are shown in table 1. Eighty-seven patients received taxane-free adjuvant chemotherapy: E/C (Epirubicin/Cyclophosphamide), 37 cases; CMF (Cyclophosphamide/Metotrexat/ 5 Fluouracil), 44 patients; others E (Epirubicin) or FEC (Epirubicin/Cyclophosphamide/5-Fluouracil), 3 cases; unknown, 3 cases.

### Protein extraction

Deep-frozen specimens of ca. 100 mg were cut from the sample and pulverized using a microdismembrator (Braun-Melsungen, Melsungen, Germany) for 2x45 sec at 200 rpm. The resulting powder was immediately suspended in 670 µl TBS with 1% Triton X-100. The suspension was incubated overnight at 4°C with gentle shaking, then subjected to ultracentrifugation for 1h at 25000 rpm at 4°C in order to remove insoluble cell material. The supernatants were stored in liquid nitrogen until use. Protein contents were determined by BCA protein assay.

### RNA isolation

For all tumors, samples were snap frozen and stored at -80°C. Tumor cell content exceeded 40% in all samples as shown by H&E staining of cryo-cut sections. Approximately 50 mg of frozen breast tumor tissue was crushed in liquid nitrogen. RLT-Buffer (QIAGEN, Hilden, Germany) was added and the homogenate was centrifuged through a QIAshredder column (QIAGEN). From the eluate, total RNA was isolated by the RNeasy Kit (QIAGEN) according to the manufacturer's instructions. RNA yield was determined by UV absorbance and RNA quality was assessed by analysis of ribosomal RNA band integrity on an Agilent 2100 Bioanalyzer RNA 6000 LabChip kit (Agilent Technologies, Palo Alto, CA).

### Microarray analysis

The Affymetrix (Santa Clara, CA) HG-U133A array and GeneChip System^{™} was used to quantify the relative transcript abundance in the breast cancer tissues. Starting from 5 µg total RNA, labeled cRNA was prepared using the Roche Microarray cDNA Synthesis, Microarray RNA Target Synthesis (T7) and Microarray Target Purification Kit, according to the manufacturer's instructions. In the analysis settings, the global scaling procedure was chosen which multiplied the output signal intensities of each array to a mean target intensity of 500. Samples with suboptimal average signal intensities (i.e., scaling factors > 25) or GAPDH 3'/5' ratios > 5 were relabeled and rehybridized on new arrays.

### Statistics

Correlations between ALCAM protein or mRNA expression and histological or clinical tumor characteristics were calculated by Chi-square tests using the SPSS 13.0 software. For this purpose, the cases were divided into three groups of similar sizes with low, moderate and high ALCAM expression. For prognostic parameters, the following groups were compared: histological grade: G1/G2 versus G3; staging: pT1 versus pT2 versus pT3/pT4; nodal involvement versus nodal-negative tumors; ER/PR positive cases versus ER/PR negative tumors; ductal versus lobular versus other carcinomas; age <56 years versus 56 years and older. Kaplan-Meier analysis was performed using GraphPad Prism^{™} 4.01 software (San Diego, CA, USA). OAS (MSS) was computed from the date of diagnosis to the date of death due to distant metastasis. Survival curves were compared with the logrank test. Univariate as well as multivariate p values for the respective risk factors in the survival model were obtained by a Cox proportional hazards model as implemented in SPSS. All tests were performed at a significance level of p=0.05. All p values are two-sided, and no corrections for multiple testing were applied.

For analysis of the other marker genes (Osteopontin, Her-2/neu, EGFR, uPA/PAI-1 and ESR 1) the afore said applies as well.

### Results:

### Correlation of ALCAM protein expression with clinical and histological parameters

For statistical analysis, the tumors were divided into three groups of similar size according to ALCAM expression levels. No significant correlations of ALCAM expression with age, menopausal status, histological tumor type, clinical stage or nodal involvement were found (data not shown), and only a weak association with histological grading which did not reach statistical significance was detected (table 2). Additionally, ALCAM protein levels were not significantly associated with expression of the progesterone receptor (PR). In contrast, there was a positive correlation of ALCAM and estrogen receptor (ER/ESR) protein levels (p=0.025; table 2).

### ALCAM RNA detection and correlation with Western blot results

For the analysis of ALCAM mRNA expression, the datasets obtained on Affymetrix chips using 162 breast cancer samples were used. Since the genechips harbored two probes for CD166/ALCAM (201951_at and 201952_at), first the expression data for both probes were compared by Spearman analysis, and a highly significant positive correlation of both results was found (p<0.0001; data not shown).

In 133 breast cancer cases ALCAM protein and RNA data were available, and subsequently correlated with each other. There was a highly significant positive correlation of ALCAM protein and ALCAM mRNA expression for both probes (p<0.0001; data not shown). Since the 201952_at probe is characterised by a higher sequence specificity compared to 209151_at, only the 201952_at results were used for further analysis.

### Correlation of ALCAM mRNA expression with established prognostic parameters

For statistical analysis, the patients were divided into three groups of similar sizes with low, moderate and high ALCAM mRNA expression levels. Similar to the results obtained for the Western blot data, no correlations with age, menopausal status, clinical stage, nodal involvement or PR status were found by Chi-square tests (data not shown). In addition, there was no significant correlation with estrogen receptor expression, and only a weak association of low ALCAM levels and higher grading which did not reach statistical significance (table 2). By scatterplot analysis of the expression data obtained with Affymetrix chips, no significant correlation of ALCAM with ESR 1 or PR mRNA levels was found (data not shown).

### Univariate survival analysis

In order to analyze the prognostic or predictive impact of ALCAM, the cohort was divided into two groups with high and absent/low ALCAM expression levels separated by the median protein or mRNA expression value. By Kaplan-Meier analysis including all cases, comparison of both groups revealed a slightly better prognosis of cases with higher ALCAM protein or RNA levels. Yet, this difference did not reach statistical significance (data not shown).

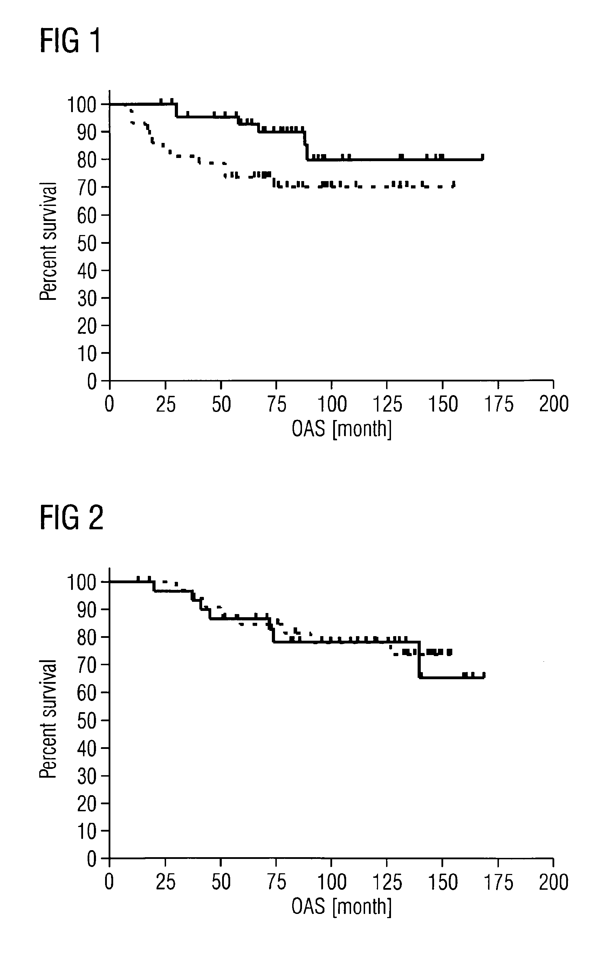

By stratified analysis of patient subgroups, striking differences were found between patients who received adjuvant chemotherapy and those who were treated with hormone therapy or radiation only. In the group treated with chemotherapy, high ALCAM protein expression was associated with longer DFS (p=0.201; data not shown) and OAS (p=0.071) (Fig. 1). This effect was not observed in patients without adjuvant chemotherapy (Fig. 2).

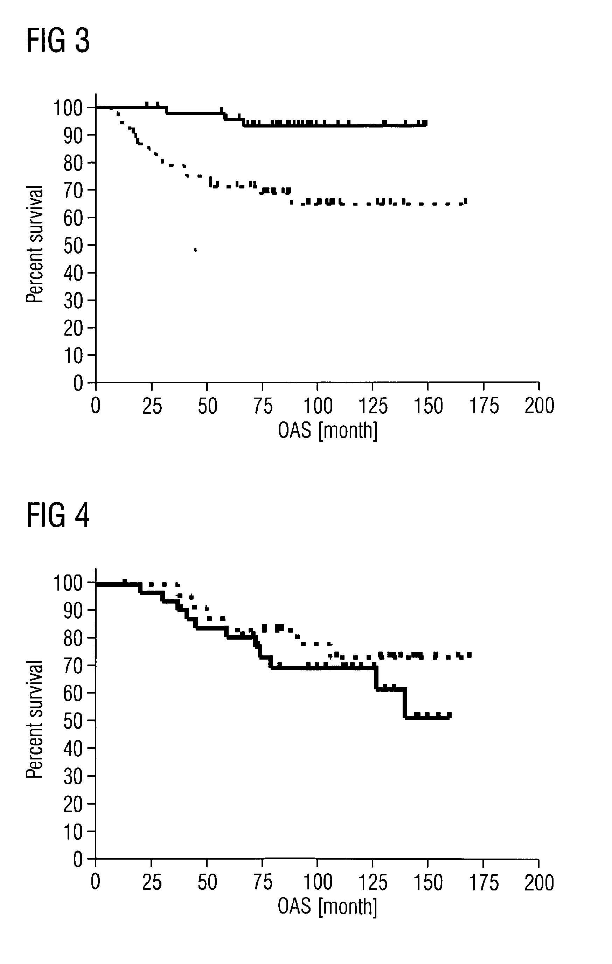

These results were confirmed and strengthened by analysis of the mRNA data: Patients with adjuvant chemotherapy and high ALCAM mRNA expression had a longer DFS (p=0.054; data not shown) and OAS (p=0.0012) than those with lower ALCAM expression (Fig. 3). In contrary, high ALCAM expression was associated with shorter disease-free survival (data not shown) and OAS in patients who did not receive adjuvant chemotherapy, but this difference was not statistically significant (Fig. 4).

For optimization of the cutoff value, the patients were further grouped in quartiles. By this approach, the prognostic impact of ALCAM expression was increased or still present in the chemotherapy group if the first quartile with lowest ALCAM expression levels (Q1) was compared to the remaining 75% of the cases (Q2-4). Using this cutoff, low ALCAM mRNA expression was strongly associated with shortened DFS (p=0.0163; data not shown) and OAS (p=0.0033; data not shown). In patients who did not get adjuvant chemotherapy, the differences observed with the median cut-off largely disappeared (data not shown).

In a stratified analysis according to endocrine therapy, not any influence of ALCAM expression on disease outcome have been found in patients who received tamoxifen for adjuvant treatment. In patients without endocrine treatment (80% of them were treated with adjuvant chemotherapy), high ALCAM mRNA expression was associated with significantly longer OAS (p=0.0258). In an analysis of our ALCAM protein expression data, this effect could not be observed (data not shown).

### Multivariate analysis

The predictive impact of ALCAM mRNA expression in patients treated with taxane-free chemotherapy was further analyzed by multivariate Cox regression analysis (forward inclusion of variables) including histological grading, nodal involvement, ESR 1 status, clinical stage and the logarithmic values of ALCAM mRNA expression. By this model, high ALCAM mRNA expression, positive ESR 1 status and absence of nodal metastasis were independent predictors of long DFS (table 3A). For OAS, ALCAM expression was the strongest independent predictive marker (p=0.001). In addition, only the ESR 1 status retained its prognostic significance by this approach, and the clinical stage was of borderline significance only (table 3B).

### Example 2

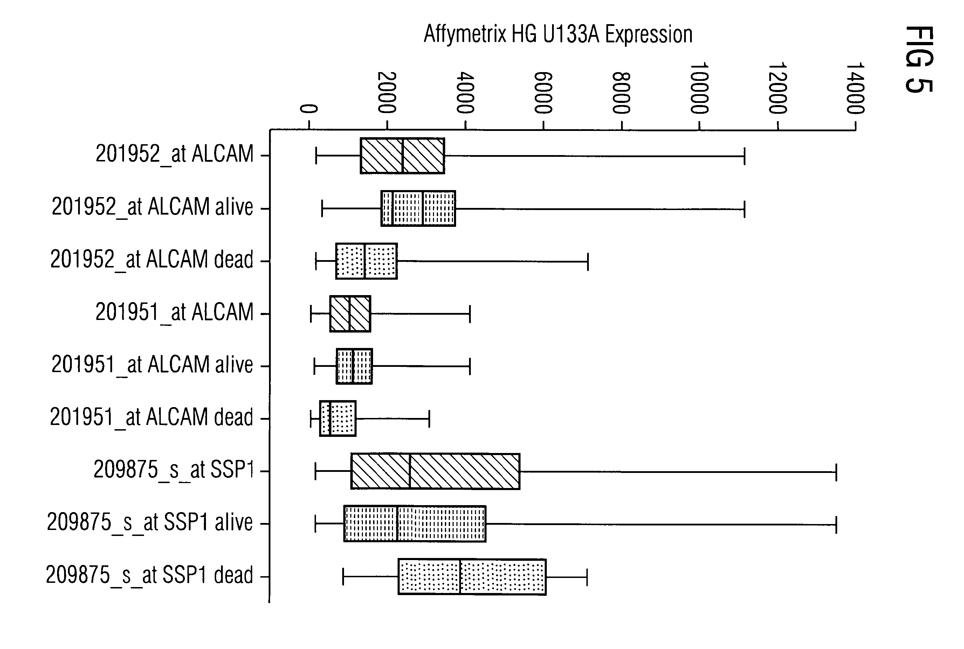

Via the Box and Whiskers analysis (Fig. 5) it could be demonstrated a positive correlation of an upregulated ALCAM mRNA expression (201951_at and 201952_at) and longer overall survival (OAS; p=0.0003 and p=0.0032) in patients treated with adjuvant chemotherapy (n=100). In contrast, it could be demonstrated a correlation of an upregulated Osteopontin (SSP1) mRNA expression (209875 _s_at) and shorter overall survival (OAS; p=0.0225) in patients treated with adjuvant chemotherapy.

These results were confirmed by Box and Whiskers analysis regarding disease free interval (data not shown).

Furthermore, via Kaplan Meier analysis a combined analysis of ALCAM and Osteopontin mRNA expression with inclusion of ESR1 mRNA expression has been performed in patients treated with adjuvant chemotherapy regarding overall survival. The survival curves are significantly different. It could be demonstrated a positive correlation of an upregulated ALCAM mRNA expression (group 1) and longer overall survival (p=0.0003), a correlation of an upregulated ESR1 mRNA expression (group 3) and shortened overall survival (compared to group 1) and a correlation of an upregulated Osteopontin mRNA expression (group 2) and still shortened overall survival (Fig. 6).

Furthermore, via Kaplan Meier analysis a combined analysis of ALCAM and Osteopontin mRNA expression with inclusion of ESR1 mRNA expression has been performed in patients treated with adjuvant chemotherapy regarding disease free interval. The survival curves are significantly different as well. It could be demonstrated a positive correlation of an upregulated ALCAM mRNA expression (group 1) and longer disease free interval (DFI; p=0.0005), a correlation of an upregulated ESR1 mRNA expression (group 3) and shortened disease free interval (compared to group 1) and a correlation of an upregulated Osteopontin mRNA expression (group 2) and still shortened disease free interval (Fig. 7).

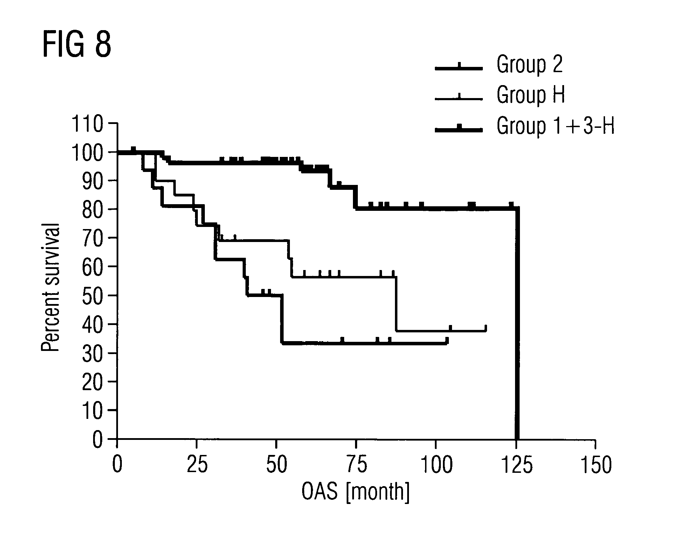

Furthermore, via Kaplan Meier analysis a combined analysis of ALCAM and Osteopontin mRNA expression with inclusion of ESR1 and Her-2/neu mRNA expression has been performed in patients treated with adjuvant chemotherapy regarding overall survival. The survival curves are significantly different as well. It could be demonstrated a positive correlation of a combination of an upregulated ALCAM mRNA expression and an upregulated ESR1 mRNA expression, but without upregulated Her-2/neu mRNA expression (group 1+3-H), and longer overall survival, a correlation of an upregulated Her-2/neu mRNA expression (group H, p=0.0006) and shortened disease free interval (compared to group 1+3-H) and a correlation of an upregulated Osteopontin mRNA expression (group 2, p<0.0001) and still shortened disease free interval (Fig. 8).

### Discussion

In the cohort of breast cancer patients studied in the present invention, no significant correlations of ALCAM expression with age, histological tumor type, grading, progesterone receptor status, clinical stage or nodal involvement was found neither based on protein data nor on ALCAM mRNA expression, which is partly in contrast to prior studies ^{15,16}. Yet, similar to the results obtained by Jezierska et al. ¹⁶, it has been found a significant correlation of ALCAM protein and ESR1 expression, suggesting that steroid receptors might contribute to the activation of ALCAM gene expression.

In the study of the present invention, unexpected results were obtained regarding the prognostic value of ALCAM expression: In the total cohort, ALCAM expression did not have a significant prognostic impact. However, in a stratified analysis striking differences between patients who received Taxane-free adjuvant chemotherapy and those who were treated with hormone or radiation therapy only have been found. In the group treated with chemotherapy, a correlation of high ALCAM protein expression and prolonged DFS and OAS was found. These results were confirmed by analysis of mRNA data showing that chemotherapy patients with high ALCAM-expression had a significantly longer DFS and OAS than patients with lower ALCAM expression in univariate and multivariate analysis. This association was found using the median as cut-off or comparing quartile 1 with the quartiles 2-4. The optimal cut-off should be evaluated in future, larger studies.

In contrast, high ALCAM expression had no or even a negative prognostic impact in patients treated with hormone or radiotherapy only. These results point to a predictive rather than a prognostic value of ALCAM expression in breast cancer patients. Although there was a strong correlation between ALCAM RNA and protein results, the associations were more significant and the differences more clear-cut regarding the RNA data.

In the group treated with chemotherapy, a correlation of high Osteopontin mRNA expression and shortened DFS and OAS could be demonstrated. In contrast, high Osteopontin expression had no prognostic impact in patients treated without chemotherapy. These results point to a predictive rather than a prognostic value of Osteopontin expression in breast cancer patients.

The inventors of the present invention have found, that expression of ALCAM and Osteopontin is particularly prognostic in high-risk patient populations receiving chemotherapy. Furthermore, combined analysis of ALCAM and Osteopontin with or without inclusion of Her-2/neu, EGFR, uPA/PAI-1 and/or ESR 1 expression data was superior.

Further, by combined analysis of ALCAM and Osteopontin with or without inclusion of the expression level data of Her-2/neu, EGFR, uPA/PAI-1 and/or ESR 1 an improved molecular test to select a more appropriate therapy can be provided.

At present, it can be only speculated about the molecular mechanisms underlying the results. Regarding ALCAM some experimental studies have shown that besides its role as an adhesion molecule, which supports homophilic and heterophilic intercellular interactions, ALCAM has additional, biochemical functions.

It remains to be proven in larger prospective studies whether ALCAM retains its predictive value and turn out as suitable predictive factors for treatment decisions, even in breast cancer patients treated with taxane-containing chemotherapy, which is a standard regimen in node positive patients at present.

By adjustment of the mRNA expression data of the marker genes (dividing the ESR1 and Her-2/neu expression data by a factor 5) of said cohort, the inventors of the present invention were for the first time in a position to identify by clustering a high-risk collective, characterized by patients having a poor prediction towards chemotherapy, but a promising prediction towards therapeutic regimen targeting the signalling pathway of receptors from the VEGF receptor and /or ligand family.

Since it is very difficult to determine those tumor types which are promoted by EGFR overexpression, the inventors of the present invention have for the first time suggested not to use the targets themselves as marker genes, but ALCAM and/or Osteopontin with or without Her-2/neu, EGFR, uPA/PAI-1 and/or ESR1 as marker genes to predict the clinical response of a patient suffering from or at risk of developing breast cancer towards a medicament related to the signalling pathway of receptors from the ErbB receptor family.

### Figures

Figures 1 and 2 demonstrate via Kaplan Meier analysis the prognostic and predictive impact regarding the overall survival of ALCAM protein expression in breast cancer patients who received adjuvant chemotherapy (Fig. 1; n=87) and patients who were treated with endocrine or radiotherapy only (Fig. 2; n=73). Full line: high ALCAM protein expression (above median). Dashed line: weak/absent ALCAM expression.

Figures 3 and 4 demonstrate via Kaplan Meier analysis the prognostic and predictive impact regarding the overall survival of ALCAM mRNA expression in breast cancer patients who received adjuvant chemotherapy (Fig. 3; n=100) and patients who were treated with endocrine or radiotherapy only (Fig. 4; n=62). Full line: high ALCAM protein expression (above median). Dashed line: weak/absent ALCAM expression.

Figure 5 demonstrates via Box and Whiskers-Plot the Affymetrix expression analysis of ALCAM and Osteopontin (SSP1) mRNA in breast cancer patients who received adjuvant chemotherapy.

Figure 6 demonstrates via Kaplan Meier analysis the prognostic and predictive impact regarding the overall survival of ALCAM, Osteopontin and ESR1 mRNA expression in breast cancer patients who received adjuvant chemotherapy. Group 1: upregulated ALCAM mRNA expression, group 2: upregulated Osteopontin mRNA expression and group 3: upregulated ESR1 mRNA expression.

Figure 7 demonstrates via Kaplan Meier analysis the prognostic and predictive impact regarding the disease free interval of ALCAM, Osteopontin and ESR1 mRNA expression in breast cancer patients who received adjuvant chemotherapy. Group 1: upregulated ALCAM mRNA expression, group 2: upregulated Osteopontin mRNA expression and group 3: upregulated ESR1 mRNA expression.

Figure 8 demonstrates via Kaplan Meier analysis the prognostic and predictive impact regarding the overall survival of ALCAM, Osteopontin, ESR1 and Her-2/neu mRNA expression in breast cancer patients who received adjuvant chemotherapy. Group 1+3-H: upregulated ALCAM and ESR1 mRNA expression without upregulated Her-2/neu mRNA expression, group H: upregulated Her-2/neu mRNA expression and group 2: upregulated Osteopontin mRNA expression.

Figure 9 demonstrates dendrograms showing clustered mRNA expression data of Osteopontin, ALCAM, ESR1 and Her-2/neu. For the left dendrogram the mRNA expression data of the dominant marker genes ESR1 and Her-2/neu were divided by a factor 5.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

| Table 1: | | | | |
|---|---|---|---|---|
| Cohort characteristics for ALCAM protein and mRNA expression analysis | | | | |
| | ALCAM protein analysis | | ALCAM mRNA analysis | |
| | n= | % | n= | % |
| *Age* | | | | |
| mean age | 56.4 y | | 56 y | |
| range | 29 - 85 y | | 29-85 y | |
| | | | | |

| Histological | | | | |
|---|---|---|---|---|
| Type | | | | |
| ductal | 112 | (70.0) | 116 | (71.6) |
| lobular | 29 | (18.1) | 24 | (14.8) |
| others | 17 | (10.6) | 19 | (11.7) |
| unknown | 2 | (1.3) | 3 | (1.9) |
| | | | | |

| Tumor stage | | | | |
|---|---|---|---|---|
| pT1 | 42 | (26.3) | 37 | (22.8) |
| pT2 | 102 | (63.8) | 109 | (67.3) |
| pT3 | 5 | (3.1) | 5 | (3.1) |
| pT4 | 7 | (4.4) | 9 | (5.6) |
| unknown | 4 | (2.5) | 2 | (1.2) |
| | | | | |

| Grading | | | | |
|---|---|---|---|---|
| G1 | 16 | (10.0) | 11 | (6.8) |
| G2 | 72 | (45.0) | 66 | (40.7) |
| G3 | 66 | (41.3) | 82 | (50.6) |
| unknown | 6 | (3.7) | 3 | (1.8) |
| | | | | |

| Lymph node involvement | | | | |
|---|---|---|---|---|
| Node positive | 36 | (22.5) | 44 | (27.2) |
| Node negative | 122 | (76.3) | 117 | (72.2) |
| unknown | 2 | (1.2) | 1 | (0.6) |

| Estrogen receptor status | | | | |
|---|---|---|---|---|
| positive | 112 | (70.0) | 114 | (70.4) |
| negative | 38 | (23.8) | 40 | (24.7) |
| unknown | 10 | (6.2) | 8 | (4.9) |
| | | | | |

| Progesterone receptor | | | | |
|---|---|---|---|---|
| status | | | | |
| positive | 91 | (56.9) | 95 | (58.6) |
| negative | 59 | (36.9) | 59 | (36.4) |
| unknown | 10 | (6.2) | 8 | (4.9) |
| | | | | |

| Therapy (multiple therapies included) | | | | |
|---|---|---|---|---|
| adjuvant chemotherapy | 87 | (54.4) | 100 | (61.7) |
| endocrine treatment | 90 | (56.3) | 90 | (55.6) |
| radiotherapy | 102 | (63.8) | 103 | 63.6) |
| | | | | |
| Follow-up Information available | 156 | (97.5) | 161 | (99.4) |
| Recurrence | 47 | (29.4) | 50 | (30.9) |
| Died of disease (DOD) | 35 | (21.9) | 40 | (24.7) |
| | | | | |
| Total number | 160 | | 162 | |

**Table 2:**

| Correlations of ALCAM protein and RNA expression with histological grading and estrogen receptor status. | | | | | |
|---|---|---|---|---|---|
| ALCAM protein analysis | | | | | |
| | | low | moderate | high | |
| | n= | (n=51) | (n=51) | (n=52) | |
| | | | | | |

| *Grading* | | | | | |
|---|---|---|---|---|---|
| G1-2 | 88 | 25 | 36 | 27 | |
| G3 | 66 | 26 | 15 | 25 | *p=0.057* |
| | | | | | |

| *estrogen receptor* | | | | | |
|---|---|---|---|---|---|
| ER positive | 112 | 30 | 42 | 40 | |
| ER negative | 38 | 19 | 8 | 11 | *p=0.025* |
| | | | | | |

| ALCAM mRNA analysis | | | | | |
|---|---|---|---|---|---|
| | | low | moderate | high | |
| | n= | (n=52) | (n=54) | (n=53) | |
| | | | | | |

| *Grading* | | | | | |
|---|---|---|---|---|---|
| G1-2 | 77 | 20 | 25 | 32 | |
| G3 | 82 | 32 | 29 | 21 | p=0.074 |
| | | | | | |

| *estrogen receptor* | | | | | |
|---|---|---|---|---|---|
| ER positive | 114 | 33 | 40 | 41 | n.s. |
| ER negative | 40 | 17 | 11 | 12 | |

| | | | | | |
|---|---|---|---|---|---|
| n.s. = not specified | | | | | |

**Table 3:**

| Cox regression analysis including convent. nostic markers and ALCAM mRNA expres- sion | | | |
|---|---|---|---|
| A. | Disease-free survival | | |

| | | | |
|---|---|---|---|
| | HR | 95% CI | p value |
| | | | |
| ALCAM | 0.308 | | 0.023 |
| ER | 0.463 | | 0.039 |
| pN | 2.335 | | 0.026 |
| pT | 1.271 | | 0.374 |
| Grading | 1.963 | | 0.113 |
| | | | |

| B. | Overall survival | | |
|---|---|---|---|
| | HR | 95% CI | p value |
| | | | |
| ALCAM | 0.092 | | 0.001 |
| ER | 0.344 | | 0.025 |
| pN | 2.169 | | 0.115 |
| pT | 1.874 | | 0.055 |
| Grading | 1.349 | | 0.529 |

**Table 4: Genes of Interest**

| Gene_ Symbol [A] | Ref. Sequences Description [A] | Ref. Sequences [A] | Unigene_ID [A] |
|---|---|---|---|
| ESR1 | Estrogen receptor | NM_000125.2 | Hs.208124 |
| EGFR / HER-1 | epidermal growth factor receptor (erythroblastic leukemia viral (v-erb-b) oncogene homolog. avian) | NM_005228 | Hs.488293 |
| ERBB2 / Her-2/neu / HER-2 | v-erb-b2 erythroblastic leukemia viral oncogene homolog 2. neuro/glioblastoma derived oncogene homolog | NM_004448 | Hs.446352 |
| ERBB2 / Her-2/neu / HER-2 | v-erb-b2 erythroblastic leukemia viral oncogene homolog 2. neuro/glioblastoma derived oncogene homolog | NM_001005862.1 | Hs.446352 |
| ALCAM | Activated leukocyte cell adhesion molecule | NM_001627.2 | Hs.591293 |
| SSP1 | Osteopontin | NM_015571.2 | Hs.485784 |
| uPA | urokinase plasminogen activator | NM_002658.2 | Hs.77274 |
| PAI-1 | plasminogen activator inhibitor-1 | NM_000602.1 | Hs.414795 |

The terms "Ref. Sequences" and "Unigene_ID" relate to databases in which the respective proteins are listed under the given access number. These databases can be accessed over the NCBI server.

**Table 5: Multivariate Analysis - Pearson Correlation**

| **Correlations** | | | |
|---|---|---|---|
| | 209875_s_al SSP1 | 201952_at ALCAM | 201951_at ALCAM |
| 209875_s_at SSP1 | 1,00 | 0,01 | 0,04 |
| 201952_at ALCAM | 0,01 | 1,00 | 0,95 |
| 201951_at ALCAM | 0,04 | 0,95 | 1,00 |
| PGR | -0,10 | 0,06 | 0,13 |
| ESR1 | -0,22 | 0,04 | 0,07 |
| ERBB2 | 0,02 | 0,22 | 0,19 |
| PLAU | 0,50 | -0,11 | -0,09 |
| PLAUR | 0,64 | -0,22 | -0,23 |
| SERPINE1 | 0,34 | -0,18 | -0,12 |
| EGFR | 0,03 | -0,06 | -0,02 |
| VEGF | 0,52 | -0,21 | -0,21 |
| VEGF1 | 0,52 | -0,19 | -0,17 |
| VEGF2 | 0,45 | -0,23 | -0,22 |
| VEGF3 | 0,47 | -0,20 | -0,20 |

### References

1. Jemal A et al., CA Cancer J Clin 2007; 57:43-66.
2. Veronesi U et al., Lancet 2005; 365:1727-1741.
3. Gesellschaft der epidemiologischen Krebsregister in Deutschland e.V. (GEKID) und RKI, Krebs in Deutschland 2006; 5.Auflage, Saarbrücken 2006.
4. van Kempen LCLT et al., J Biol Chem 2001; 176: 25783-90.
5. Swart GWM, Eur J Cell Biol 2002; 81: 313-21.
6. Bruder SP et al., J Bone Mineral Res 1998; 13: 655-63.
7. Bowen MA and Aruffo A, Transplant Proc 1999; 31: 795-6.
8. Fujiwara H et al., J Clin Endocrinol Metab 2003; 88: 3437-43.
9. Ohneda O et al., Blood 2001; 98: 2134-42.
10. King J et al., Chest 2004; 125: 150S-151S.
11. Swart GWM et al., Cancer Met Rev 2005; 24: 223-36.
12. Kristiansen G et al., Prostate 2003; 54: 34-43.
13. Weichert W et al., J Clin Pathol 2004; 57: 1160-4.
14. Verma A et al., Oncology 2005; 68: 462-70.
15. King JA et al., Breast Cancer Res 2004; 6: R478-87.
16. Jezierska A et al., Med Sci Monit 2006; 12: BR245-56.
17. Burkhardt M et al., J Clin Pathol 2006; 59: 403-9.
18. Tuck AB et al., Int J Cancer 1998; 79: 502-8.
19. Rudland PS et al., Cancer Res 2002; 62: 3417-27.
20. Bramwell VH et al., Clin Cancer Res 2006; 12(11 Pt 1): 3337-43.
21. Wang-Rodriguez J et al., Breast Cancer Res 2003; 5(5): R136-43.
22. Das R et al., J Biol Chem 2004; 279(12): 11051-64.
23. Shepherd FA, N Engl J Med 2005; 353(2):123-32.

## Claims

1. A method for predicting a clinical response of a patient suffering from or at risk of developing gynecologic cancer towards a given mode of treatment, said method comprising the steps of:
a) obtaining a biological sample from said patient;
b) determining the expression level of at least one gene selected from the group comprising ALCAM, Osteopontin, Her-2/neu, EGFR, uPA/PAI-1 and/or ESR1, in particular of ALCAM and/or Osteopontin, in said sample;
c) comparing the pattern of expression level(s) determined in (b) with one or several reference pattern(s) of expression levels; and
d) predicting therapeutic success for said given mode of treatment in said patient or implementing therapeutic regimen in said patient from the outcome of the comparison in step (c).

2. The method according to claim 1, **characterized in that**
a) upregulated expression of ALCAM is indicative of a good prediction as regards therapeutic success for patients receiving a chemotherapy,
b) downregulated expression of ALCAM is indicative of a poor prediction as regards therapeutic success for patients receiving a chemotherapy,
c) upregulated expression of Osteopontin, Her-2/neu, EGFR and/or ESR1 is indicative of a poor prediction as regards therapeutic success for patients receiving a chemotherapy, and/or
d) downregulated expression of Osteopontin, Her-2/neu, EGFR and/or ESR1 is indicative of a good prediction as regards therapeutic success for patients receiving a chemotherapy.

3. The method according to any one of claims 1 to 2, wherein upregulated expression of Osteopontin and/or EGFR and/or downregulated expression of ALCAM, Her-2/neu, uPA/PAI-1 and/or ESR1 is indicative of a promising prediction as regards therapeutic success for patients receiving a therapeutic regimen targeting the signalling pathway of receptors from the VEGF receptor and /or ligand family.

4. The method according to any one of claims 1 to 3, **characterized in that** the mode of treatment for which prediction is sought is a treatment related to the signalling pathway of receptors from the ErbB receptor family.

5. The method according to any one of claims 1 to 4, **characterized in that** the mode of treatment for which prediction is sought is a treatment related to the signalling pathway of receptors from the VEGF receptor and /or ligand family.

6. The method according to any one of claims 1 to 5, wherein said given mode of treatment (a) acts on recruitment of lymphatic vessels, cell proliferation, cell survival and/or cell motility, and/or b) comprises administration of a chemotherapeutic agent.

7. The method according to any of claims 1 to 6, wherein said given mode of treatment comprises chemotherapy, in particular adjuvant chemotherapy, administration of small molecule inhibitors, antibody based regimen, anti-proliferation regimen, pro-apoptotic regimen, pro-differentiation regimen, radiation and/or surgical therapy.

8. A method of selecting a therapy modality for a patient afflicted with gynecologic cancer said method comprising the steps of:
a) obtaining a biological sample from said patient;
b) predicting from said sample, by the method according to any one of claims 1 to 7, therapeutic success for a plurality of individual modes of treatment; and
c) selecting a mode of treatment which is predicted to be successful in step (b).

9. A method of determining the prognosis for a patient afflicted with gynecologic cancer and receiving chemotherapy, said method comprising the steps of:
a) obtaining a biological sample from said patient;
b) combining the expression level data of at least two genes selected from the group comprising ALCAM, Osteopontin, Her-2/neu, EGFR, uPA/PAI-1 and/or ESR1 in said sample;
c) comparing the pattern of expression level(s) determined in (b) with one or several reference pattern(s) of expression levels; and
d) determining the prognosis in said patient from the outcome of the comparison in step (c).

10. The method according to claim 9, **characterized in that**
a) upregulated expression of ALCAM is indicative of a good prognosis for patients receiving a chemotherapy,
b) downregulated expression of ALCAM is indicative of a poor prognosis for patients receiving a chemotherapy,
c) upregulated expression of Osteopontin, Her-2/neu, EGFR and/or ESR1 is indicative of a poor prognosis for patients receiving a chemotherapy, and/or
d) downregulated expression of Osteopontin, Her-2/neu, EGFR and/or ESR1 is indicative of a good prognosis for patients receiving a chemotherapy.

11. The method according to any one of claims 1 to 10, wherein determining the expression level comprises:
a) determining the RNA expression level; and/or
b) determining the protein expression level.

12. The method according to any one of claims 1 to 11, wherein the expression level is determined by
a) a hybridization based method;
b) a PCR based method;
c) determining the protein level; and/or by
d) an array based method.

13. The method according to any one of claims 1 to 12, **characterized in that** the expression level of at least one of the said marker genes is determined with rtPCR (reverse transcriptase polymerase chain reaction) of the related mRNA.

14. The method according to any one of claims 1 to 13, **characterized in that** the expression level of at least one of the said marker genes is determined in formalin and/or paraffin fixed tissue samples.

15. The method according to any one of claims 1 to 14, wherein, after lysis, the samples are treated with silica-coated magnetic particles and a chaotropic salt, in order to purify the nucleic acids contained in said sample for further determination.

16. The method according to any one of claims 1 to 15, wherein said treatment related to the signalling pathway of receptors from the ErbB receptor family comprises the administration of an agonist of the ErbB receptor domain.

17. The method according to any one of the aforementioned claims **characterized in that** said gynecologic cancer is selected from the group comprising breast cancer, ovarian cancer, vulvar cancer, vaginal cancer, tubal cancer, endometrian cancer and/or cervical cancer.

18. The method according to any one of the aforementioned claims **characterized in that** said gynecologic cancer is breast cancer.

19. The method according to any one of the aforementioned claims, wherein said treatment related to the signalling pathway of receptors from the ErbB receptor family comprises the administration of a tyrosin kinase inhibitor.

20. A kit useful for carrying out a method of any one of the preceding claims, comprising at least
a) a primer pair and/or a probe each having a sequence sufficiently complementary to a gene encoding for ALCAM, Osteopontin, Her-2/neu, EGFR, uPA/PAI-1 and/or ESR1; and/or
b) an antibody directed against ALCAM, Osteopontin, Her-2/neu, EGFR, uPA/PAI-1 and/or ESR1.

21. Use of
a) an antibody directed against a ligand from the VEGF family,
b) an antisense nucleic acid or a ribozyme inhibiting the expression of a gene encoding for a ligand from the VEGF family, or
c) an inactive version of a ligand from the VEGF family
as an antagonist for the preparation of a pharmaceutical composition for the treatment of said high-risk breast cancer patients identified by a method according to any one of the preceding claims.

22. A method for correlating the clinical outcome of a patient suffering from or at risk of developing gynecologic cancer with the presence or non-presence of a defect in expression of at least one gene selected from the group comprising ALCAM, Osteopontin, Her-2/neu, EGFR, uPA/PAI-1 and/or ESR1, said method comprising the steps of:
a) obtaining a (fixed) biological sample from said patient;
b) determining the expression level of at least one of the said marker genes in said patient according to any of the above methods; and
c) correlating the pattern of expression level(s) determined in (b) with said patient's data, said data being selected from the group consisting of etiopathology data, clinical symptoms, anamnesis data and/or data concerning the therapeutic regimen.

23. A nucleic acid primer pair and/or a nucleic acid probe having a sequence sufficiently complementary to a gene encoding for ALCAM, Osteopontin, Her-2/neu, EGFR, uPA/PAI-1 and/or ESR1.

24. An antibody directed against ALCAM, Osteopontin, Her-2/neu, EGFR, uPA/PAI-1 and/or ESR1.
